# EUROPÄISCHE PATENTSCHRIFT

(11) **EP 2 445 437 B1**
(45) Veröffentlichungstag und Bekanntmachung des Hinweises auf die Patenterteilung: **23.11.2016**
(21) Anmeldenummer: 10728138.8
(22) Anmeldetag: 25.06.2010
(51) Int. Cl.: A61B 34/20

(54) **VERFAHREN ZUM ERZEUGEN VON POSITIONSDATEN EINES INSTRUMENTES**
METHOD FOR GENERATING POSITION DATA OF AN INSTRUMENT
PROCÉDÉ DE PRODUCTION DE DONNÉES DE POSITION D'UN INSTRUMENT

(30) Priorität: 26.06.2009 DE 102009030731
(43) Veröffentlichungstag der Anmeldung: 02.05.2012
(73) Patentinhaber: Fiagon GmbH, 10437 Berlin (DE)
(72) Erfinder: MUCHA, Dirk, 10961 Berlin (DE)
(74) Vertreter: Bobbert & Partner Patentanwälte PartmbB
(86) Internationale Anmeldenummer: PCT/EP2010/003822
(87) Internationale Veröffentlichungsnummer: WO 2010/149371

(56) Entgegenhaltungen:
- EP-A1- 1 380 266
- WO-A1-99/32033
- WO-A2-2008/110553
- WO-A2-2010/054646
- US-A1- 2008 121 703
- "Computer Assisted ENT Surgery" INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE LNKD- DOI:10.1007/S11548-006-0020-1, Bd. 1, Nr. 7, 30. Mai 2006 (2006-05-30), Seiten 311-323, XP019416252 ISSN: 1861-6429 in der Anmeldung erwähnt

## Beschreibung

Die Erfindung bezieht sich auf ein Verfahren zum Erzeugen von Positionsdaten eines Instrumentes. Ferner ist die Erfindung auf ein System sowie ein Computerprogrammprodukt mit Programmcode gerichtet.

### Hintergrund der Erfindung

Beim Arbeiten mit einem Instrument in einem Bereich, der nicht direkt einsichtbar ist, ist es von Bedeutung, die aktuelle Lage des Instrumentes erfassen, um die erfassten Lage- oder Positionsdaten in geeigneter Weise weiterverarbeiten zu können. Bekannt ist, ein Instrument mit elektromagnetischen Lagesensoren zu versehen und ferner einen Feldgenerator vorzusehen, so dass eine Steuerungseinheit, die mit dem Feldgenerator und dem oder den Lagesensoren verbunden ist, Lagedaten des Instrumentes zu erfassen und auf einer Anzeigevorrichtung zur Darstellung zu bringen. Aus dem Aufsatz "Plausibility check for error compensation in electromagnetic navigation in endoscopic sinus surgery" von Dirk Mucha et al., veröffentlicht als Proc. Computer Assisted Radiology and Surgery CARS, P. 3 17- 18, 2006, ist bekannt, ein Instrument mit zwei Lagesensoren zu versehen. Um ein Problem einer Feldstörung durch metallische Geräte zu behandeln, ist bekannt, eine Plausibilitätsprüfumg von gemessenen Lagemessdaten vorzunehmen. Allerdings ist kein Weg bekannt, zusätzlich Fehler im Zusammenhang mit Bewegungen in geeigneter Weise zu behandeln.

Aus der nachveröffentlichten WO 2010/054646 A2 ist ein Verfahren zum Erzeugen von Bilddaten für eine Darstellung eines Instruments auf einer Anzeigevorrichtung bekannt. Aus der EP 1 380 266 A1 ist ein System zum Überwachen der Position eines medizinischen Instruments in Bezug auf den Körper eines Patienten und zum Anzeigen von Bildern des Körpers des Patienten in Bezug auf die Position des medizinischen Instruments bekannt, wobei aus den Positionsdaten die Geschwindigkeit des Instruments bestimmt wird. Die Positionsdaten werden ignoriert, wenn die Geschwindigkeit über einer bestimmten Schwelle liegt.

Aus der US2008/121703 A1 ist Tracking-System zur Instrumentennavigation bekannt.

Aus der WO 99/32033 A1 ist ein medizinisches System zur Anwendung an Patienten bekannt, das unter anderem mehrere Messwertaufnehmer zur Bestimmung eines medizinischen Geräts in einem Patienten aufweist.

Aus der Literaturstelle "Computer Assisted ENT Surgery" INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE LNKD-DO1:10.1007/S11548-006-0020-1, Bd. 1, Nr. 7, 30. Mai 2006 (2006-05-30), Seiten 311-323, XP019416252ISSN: 1861-6429 ist eine Auswertung aus der navigierten Chirurgie im Bereich Nase und Nasennebenhöhlen bekannt.

Aus der WO 2008/110553 A2 ist ein Verfahren zur Positionsbestimmung eines medizinischen Instruments, insbesondere einer Endoskopiekapsel, bekannt.

### Zusammenfassung der Erfindung

Aufgabe der Erfindung ist es, ein weiteres Verfahren zum Erzeugen von Positionsdaten eines Instrumentes zu schaffen.

Diese Aufgabe wird erfindungsgemäß durch ein Verfahren nach dem unabhängigen Anspruch 1 gelöst. Weiterhin ist ein System für die Ausführung solch eines Verfahrens nach dem unabhängigen Anspruch 7 und ein Computerprogrammprodukt mit Programmcode nach den unabhängigen Anspruch 10 vorgesehen. Das Verfahren umfasst die folgenden Schritte:
Erfassen von Lagemessdaten von wenigstens einem an dem Instrument angeordneten Lagesensor, Bestimmen eines Bewegungszustandes des Instrumentes auf der Grundlage der erfassten Lagemessdaten, Aufstellen von Kriterien mit Hilfe von berechneten Größen aus aufeinanderfolgende Lagemessdaten werden, um Bewegungszustände zu unterscheiden, wobei Bewegungszustände wenigstens Translationen und Rotationen umfassen, Filtern der erfassten Lagemessdaten auf der Grundlage des bestimmten Bewegungszustandes, Bereitstellen eines Bilddatensatzes für eine Darstellung des Instruments auf einer Anzeigevorrichtung wenigstens auf der Grundlage der gefilterten Lagemessdaten, welcher konfiguriert ist, das Instrument auf der Anzeigevorrichtung darzustellen, wobei der Bilddatensatz ein Bilddatensatz aus wenigstens einem Verfahren der Gruppe ist, welche umfasst: Computertomographie, digitale Volumentomographie, Magnetresonanztomographie, Sonographie, Duplexuntersuchung, Angiographie, nuklearmedizinische Bildgebungsverfahren, mit Kontrastmittelgabe einhergehende medizinische Bildgebungsverfahren, Durchleuchtungsverfahren und radiologische Additions- oder Subtraktionsverfahren und Bereitstellen des Bilddatensatzes für eine geänderte Darstellung des Instruments wenigstens auf der Grundlage der gefilterten Lagemessdaten, welcher konfiguriert ist, das Instrument darzustellen. Als Lage eines Werkzeuges wird im Folgenden wenigstens der Ort des Instrumentes in Bezug auf ein bestimmtes Koordinatensystem und/oder wenigstens eine Orientierung des Instrumentes in diesem Koordinatensystem bezeichnet. Als Lagemessdaten werden im Folgenden insbesondere Feldstärkedaten von Lagesensoren bezeichnet, die dazu geeignet sind, wenigstens eine Position und/oder wenigstens eine Orientierung des Lagesensors und des Instrumentes, an welchem der Sensor befestigt ist, bereitzustellen. Ein Lagesensor kann beispielsweise als elektromagnetischer Sensor ausgeführt sein, beispielsweise als eine Spule oder ein Satz von Spulen, aber andere Einrichtungen, die dazu in der Lage sind, eine Orts- und/oder Lageinformation auszugeben sind auch möglich. Mit anderen Worten ist ein Verfahren bereitgestellt, mittels dessen die Position eines Instruments ermittelt werden kann, ohne dass ein Benutzer des Instrumentes einen direkten Sichtkontakt zu dem Instrument haben muss.

Bevorzugte Weiterbildungen des erfindungsgemäßen Verfahrens sind jeweils Gegenstand der Unteransprüche.

Erfindungsgemäß wird die Position eines Instruments auf einer Anzeigevorrichtung dargestellt, wobei die Darstellung des Instruments wenigstens auf der Grundlage von gefilterten Lagemessdaten vorgenommen wird, die aufgrund eines Bewegungszustands gefiltert wurden.

Eine bevorzugte Weiterbildung der Erfindung sieht vor, dass die erfassten Lagemessdaten auf der Grundlage wenigstens einer aktuellen Lagemessung gebildet werden. Somit liegen Daten, die die aktuelle Lage des Instruments repräsentieren, beispielsweise in einer Steuerung, vor und können beispielsweise bearbeitet werden.

Bei einer zweckmäßigen Ausgestaltung der Erfindung kann vorgesehen sein, dass die erfassten Lagemessdaten eine Mehrzahl von Lagemessungen umfassen. Dies wird beispielsweise durch einen Speicher ermöglicht, der in einer Steuerungsvorrichtung vorgesehen ist, welche dazu hergerichtet ist, das erfindungsgemäße Verfahren auszuführen. Die Mehrzahl von Lagemessungen kann beispielsweise innerhalb konstanter Zeitintervalle aufgenommen sein, so dass eine Messreihe von Lagemessungen vorliegt.

Die Erfindung sieht vor, dass ein Plausibilitätswert auf der Grundlage von Lagemessdaten gebildet wird, die unter Verwendung von wenigstens zwei an dem Instrument angeordneten Lagesensoren erfasst werden.

Jeder der an dem Instrument angeordneten Lagesensoren kann dazu konfiguriert sein, Lagemessdaten auszugeben, aus denen eine Lage des Instrumentes bestimmt werden kann. Insbesondere kann mittels geeigneter Transformationen beispielsweise ein Instrumentenarbeitspunkt bestimmt werden. Idealerweise führen die Auswertungen der Lagemessdaten aller an dem Instrument angeordneten Lagesensoren zu demselben Instrumentenarbeitspunkt. Befindet sich allerdings ein leitender, beispielsweise metallischer, Gegenstand in der Nähe eines der Lagesensoren, so wird die Feldstruktur in der Nähe dieses Lagesensors verändert, so dass bei der Auswertung der Lagemessdaten dieses Lagesensors ein anderer Instrumentenarbeitspunkt ermittelt wird als bei der Auswertung der Lagemessdaten des weitestgehend ungestörten Lagesensors. Der Betrag des Abstandes zwischen den beiden ermittelten Instrumentenarbeitspunkten wird als Plausibilitätswert bezeichnet. Ein großer Plausibilitätswert bedeutet also eine große Abweichung der bestimmten Instrumentenarbeitspunkte. Ein auf diese Weise verursachter Fehler wird als Fremdstörung bezeichnet.

Erfindungsgemäß wird der Bewegungszustand auf der Grundlage von sowohl aktuell erfassten als auch in der Vergangenheit erfassten Lagemessdaten bestimmt. Unterschiedliche Bewegungen des Instrumentes, wie beispielsweise Translationen oder Rotationen oder Kombinationen dieser Bewegungen mit großer oder kleiner Geschwindigkeit können auf diese Weise ausgewertet und kategorisiert und schließlich einem Bewegungszustand zugeordnet werden.

Erfindungsgemäß ist vorgesehen, dass jedem Bewegungszustand ein Plausibilitätsschwellwert zugeordnet ist. Auf diese Weise ist es mit anderen Werten möglich, auf der Grundlage vergangener Bewegungen des Instrumentes einen Plausibilitätsschwellwert für eine weitere Auswertung festzulegen.

Es ist erfindungsgemäß vorgesehen, dass das Filtern der erfassten Lagemessdaten auf der Grundlage wenigstens eines Vergleiches des Plausibilitätswertes mit dem Plausibilitätsschwellwert erfolgt. Da der Plausibilitätswert von einer Feldstörung durch einen leitfähigen, beispielsweise metallischen, Körper in der Nähe eines der Lagesensoren beeinflusst wird und der Plausibilitätsschwellwert auf der Grundlage des Bewegungszustandes des Instrumentes bestimmt wird, wird eine Bewegung des Instrumentes in einem gestörten Feld betrachtet, so dass diese unterschiedlichen Fehlerquellen letztendlich mit in die Positionsberechnung einfließen können. Hieraus kann sich eine Darstellung des Instrumentes auf der Anzeigevorrichtung ergeben.

Bevorzugt sieht eine Fortbildung der Erfindung vor, dass das Filtern ein Akzeptieren der aktuellen Lagemessdaten für den Fall, dass der Plausibilitätswert kleiner oder gleich dem Plausibilitätsschwellwert ist, und ein Verwerfen der aktuellen Lagemessdaten für den Fall, dass der Plausibilitätswert größer als der Plausibilitätsschwellwert ist, umfasst. Ist also ein Plausibilitätswert größer als ein Plausibilitätsschwellwert, so wird die entsprechende aktuelle Lage verworfen. Auf diese Weise werden für einen Benutzer verwirrende Positionswertänderungen vermieden.

Bei einer zweckmäßigen Ausgestaltung der Erfindung kann vorgesehen sein, dass der Bilddatensatz auf der Grundlage von wenigstens aktuellen Lagemessdaten und dem Plausibilitätswert gebildet wird. Auf der Grundlage der aktuellen Lagemessdaten wird vorzugsweise der Instrumentenarbeitspunkt ermittelt, und der Plausibilitätswert kann beispielsweise als Radius einer um den Instrumentenarbeitspunkt beschriebenen Kugel ausgewertet werden. Auf diese Weise kann für ein nachgeschaltetes Verfahren oder für nachfolgende Berechnungen eine Unsicherheit des aktuellen Instrumentenarbeitspunktes berücksichtigt werden (z.B. kein auch ein Alarm ausgegeben werden). Bei entsprechender Ausgestaltung kann dem Benutzer wie beispielsweise dem operierenden Arzt die Unsicherheit des aktuellen Instrumentenarbeitspunktes alternativ oder zusätzlich mitgeteilt und/oder visualisiert werden.

Bei einer zweckmäßigen Ausgestaltung der Erfindung kann vorgesehen sein, dass auf der Grundlage des Plausibilitätswertes und der erfassten Lagemessdaten bestimmt wird, ob oder ob nicht eine Eigenstörung und / oder eine Fremdstörung der erfassten Lagemessdaten vorliegt.

Bevorzugt sieht eine Fortbildung der Erfindung vor, dass die Fremdstörung auf der Grundlage des Plausibilitätswertes und die Eigenstörung auf der Grundlage der erfassten Lagemessdaten bestimmt werden. Als Fremdstörung wird insbesondere eine Störung angesehen, die durch einen leitfähigen, beispielsweise metallischen, Gegenstand zu Lagemessdatensätzen der Lage-Sensoren führt, auf deren Grundlage abweichende Instrumentenarbeitspunkte ermittelt werden. Als Eigenstörung wird eine Bewegung, beispielsweise ein Positionieren, des Instrumentes angesehen. Das bedeutet, dass die gemessene Instrumentenposition nicht oder durch unzureichende Kalibrierdaten beeinflusst wird. Eine Eigenstörung kann auch ein Rotieren des Instrumentes sein. Das bedeutet, dass die gemessene Instrumentenposition durch die Rotationsbewegung beeinflusst wird. Eine Eigenstörung kann ferner auch ein Bewegen des Instrumentes umfassen. Das bedeutet, dass die gemessene Instrumentenposition durch die Translationsbewegung beeinflusst wird.

Gemäß einem weiteren Aspekt der Erfindung ist ein System zur Ermittlung der Positionsdaten eines Instrumentes, insbesondere eines medizinischen Instrumentes, und/oder zur Darstellung des Instruments oder von Abschnitten hiervon auf eine Anzeigevorrichtung bereitgestellt, wobei das System umfasst: Wenigstens einen an dem Instrument angeordneten Lage-Sensor zum Erfassen von Lagemessdaten des Instrumentes und eine Datenverarbeitungseinrichtung, die dazu konfiguriert ist, das erfindungsgemäße Verfahren auszuführen. Das System ist geeignet und konfiguriert, einen auf der Grundlage des zuvor beschriebenen Verfahrens bestimmten Bilddatensatz darzustellen. In einer wiederum bevorzugten erfindungsgemäßen Ausführungsform weist das System ein Betriebssystem auf. Das Betriebssystem ist vorzugsweise ein Unix- Betriebssystem, wie z.B. Solaris, UnixWare oder IRIX. oder ein Unix-Derivat bzw. BSD-Betriebssystem wie z.B. Linux, NetWare, GNU/Hurd, FreeBSD oder NetBSD. Das Betriebssystem ist weiter bevorzugt ein auf einem Apple- oder Apple/Mc-Instosh-Computer lauffähiges Betriebssystem wie z.B. Mac OS oder Mac OS X. Das Betriebssystem kann des Weiteren bevorzugt ein Produkt der Firma Microsoft sein, z.B. Windows in jedem dem Fachmann geläufigen Typ oder Version, z.B. Windows XP, Window Me, Windows 2000, Windows Vista, Windows 7, Windows CE, Windows NT usw.

In einer weiter bevorzugten Ausführungsform werden Bilddaten zu anatomischen Gegebenheiten oder zur Morphologie zugeordnet. Dies kann beispielsweise mittels oberflächenbasierter Registrierungsverfahren oder einer Punktpaarregistrierung erfolgen.

Ein weiterer Aspekt der Erfindung sieht ein Computerprogramm-Produkt mit Programmcode vor, der dazu konfiguriert ist, das beschriebene Verfahren auszuführen, wenn der Programmcode auf einem Computer ausgeführt wird.

Der Programmcode liegt bevorzugt in der Programmiersprache C, C++, C#, Java, Delphi, Ada, Fortran, Cobol oder in einer Interpreter bzw. Scriptsprache wie Javascript, Python, Perl, Python, Lua, Ruby oder Matlab-Script vor.

Gemäß einem weiteren Aspekt ist ein Datenträger mit Programmcode bereitgestellt, der dazu konfiguriert ist, das beschriebene Verfahren auszuführen, wenn der Programmcode auf einem Computer ausgeführt wird.

Das erfindungsgemäße Verfahren sowie das System, das Computerprogrammprodukt und das Computerprogramm und der Datenträger mit Programmcode ermöglichen in der oben geschilderten Weise einem Benutzer eines Instrumentes eine sichere Kenntnis der Positionsdaten und bei Belieben zudem eine sichere Instrumentenführung, sogar, wenn er keinen Sichtkontakt zu dem Instrument hat. Das Instrument kann beispielsweise ein Zeiger, Bohrer, Führungsdraht, Katheter, Endoskop, Biopsienadel, Schneidinstrument oder jegliches andere Instrument, insbesondere auf dem medizinischen Gebiet, sein.

Unabhängig von dem oben beschriebenen Verfahren, System, Computerprogrammprodukt, Computerprogramm und Datenträger ist ein wenigstens einen Navigationsmodul und einen Monitor umfassendes Endoskopiesystem bereitgestellt, das dazu konfiguriert ist, wenigstens eine synchronisierte Video- und Navigationsinformation auf dem Monitor, insbesondere Endoskopiemonitor, bereitzustellen. Vorzugsweise ist das Endoskopiesystem dazu konfiguriert, eine auf der Grundlage des oben beschriebenen Verfahrens synchronisierte Video- und Navigationsinformation auf dem Monitor darzustellen. Die Synchronisation umfasst beispielsweise eine Aktualisierung der, wie oben beschrieben, um den Instrumentenarbeitspunkt herum dargestellten, auf der Grundlage des Plausibilitätswertes und der erfassten Lagemessdaten bestimmten Kugel zur Veranschaulichung einer Messunsicherheit. Die Aktualisierung der Darstellung kann beispielsweise auf einer Aktualisierung des jeweils zur Anzeige gebrachten Bilddatensatzes beruhen.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung naher erläutert. Hierbei zeigen:
- Fig. 1: in schematischer Darstellung ein Instrument mit zwei Lagesensoren und Koordinatensystemen,
- Fig. 2: ein Instrument wie in Fig. 1 mit einem Störkörper,
- Fig. 3: ein Konzept der adaptiven Schwellwertfilterung,
- Fig. 4: ein Konzept einer adaptiven Schwellwertfilterung wie in Fig. 3 mit einer Erweiterung,
- Fig. 5: einen Klassifizierungsalgorithmus zum Bestimmen eines Bewegungszustandes des Instrumentes,
- Fig. 6: einen schematischen Aufbau eines Endoskopiesystems mit Endoskopiemonitor und
- Fig. 7: einen Endoskopiemonitor gemäß Fig. 6.

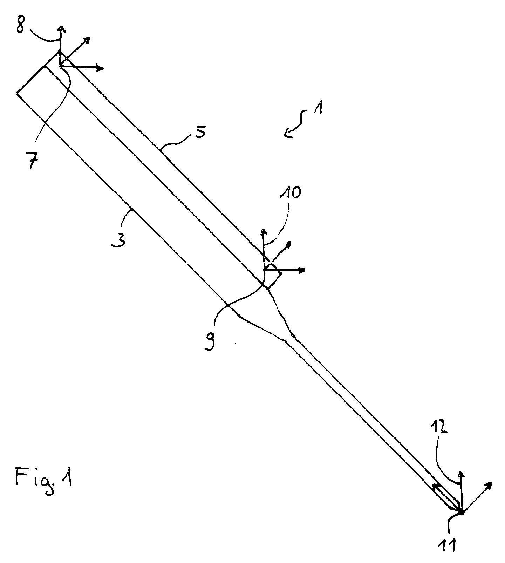

Fig. 1 zeigt ein Instrument 1, insbesondere ein medizinisches Instrument 1, an dem ein Lokalisator 5 fest angeordnet ist, an welchem zwei Lagesensoren 7 und 9 angebracht sind. Das Instrument 3 ist in diesem Beispiel mit einer Spitze 11 versehen, die den Instrumentenarbeitspunkt darstellt. Den relevanten Punkten sind lokale Koordinatensysteme zugeordnet: Dem Lagesensor 7 ist ein Koordinatensystem 8 mit Ursprung 7 zugeordnet, dem Lagesensor 9 ist ein Koordinatensystem 10 mit Ursprung 9 zugeordnet und dem Instrumentenarbeitspunkt 11 ist ein Koordinatensystem 12 mit Ursprung 11 zugeordnet. Die Lagesensoren 7 und 9 erfassen ein von einer oder mehreren Feldspulen erzeugtes elektromagnetisches Feld. Die Messwerte der Lagesensoren 7 und 9 werden an eine Steuereinrichtung (hier nicht gezeigt) weitergeleitet. Die Steuereinrichtung ist dazu konfiguriert, aus den Lagemessdaten des jeweiligen Lagesensors 7 und 9 eine Lage der lokalen Koordinatensysteme 8 und 10 zu berechnen. Mit Lage ist hier insbesondere der Ort in einem dreidimensionalen Koordinatensystem wie auch die Orientierung des jeweiligen Lagesensors 7 oder 9 gemeint. Aus der bekannten Geometrie des Instrumentes 3 kann im idealen Fall bereits aus den Lageangaben eines einzelnen Lagesensors die Lage des Instrumentenarbeitspunktes 11, also Ursprung und Orientierung des Koordinatensystems 12, berechnet werden.

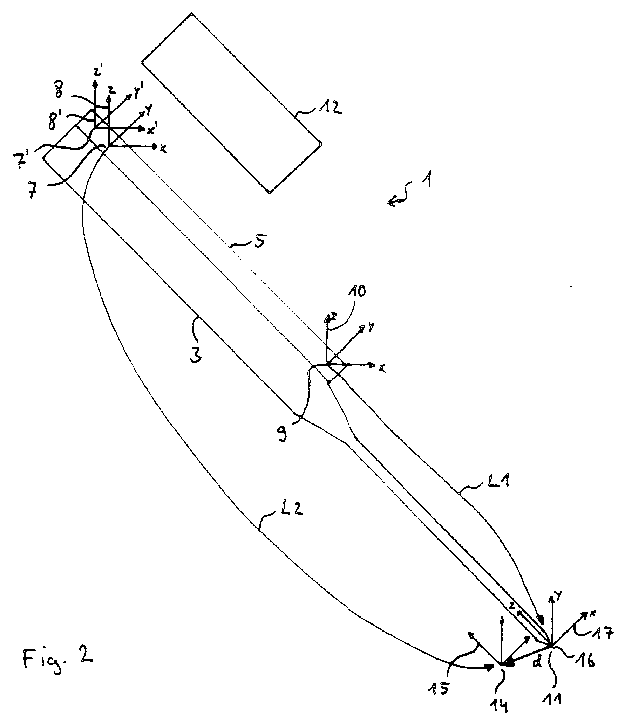

Fig. 2 zeigt das Instrument aus Fig. 1, wobei die gleichen Bezugszeichen gleiche Komponenten bedeuten. In dem in Fig. 2 dargestellten Fall befindet sich ein störendes Objekt 12 in der Nähe des Instrumentes 3. Das Objekt 12 ist in der Weise störend, dass es in der Lage ist, die durch die nicht gezeigte Feldspule oder Feldspulen erzeugten Felder derart zu verändern, dass die aus den Lagesensoren 7 und 9 ausgelesenen Signale in unerwünschter Weise ausgewertet werden. Dies führt dazu, dass die Auswertung der Lagemessdaten des Lagesensors 9, welcher einen großen Abstand zum Störobjekt 12 aufweist, in der Steuereinheit als ein Ergebnis für die Lage des Instrumentenarbeitspunktes ein Koordinatensystem 17 mit einem Ursprung 16 ermittelt, während die Auswertung der Lagemessdaten des Lagesensors 7 als Ergebnis ein Koordinatensystem 15 mit einem Ursprung 14 liefert. Die Abweichung zwischen den Ursprüngen 16 und 14 der Koordinatensysteme 17 und 15 wird als d bezeichnet. Der Betrag dieses Vektors d wird als Plausibilitätswert bezeichnet und wird im Folgenden weiter verwendet. Der Steuereinheit der Auswertelogik liegt keine Information vor, welcher der Lage-Sensoren stärker gestört wird. Daher ist die Steuereinheit nicht dazu in der Lage, zu entscheiden, welches der Koordinatensysteme 15 mit Ursprung 14 oder 17 mit Ursprung 16 den Instrumentenarbeitspunkt besser darstellt. Zur Darstellung auf einer Anzeigevorrichtung wird daher die Instrumentenspitze mit einem Kreis einer Kugel versehen, deren Durchmesser dem Plausibilitätswert entspricht, so dass ein Benutzer eines derartigen Systems in der Lage ist, auf der Grundlage dieser Bildschirmdarstellung zu entscheiden, wie genau oder ungenauer im Augenblick gerade das Instrument im Raume anordnet. Ein weiteres Problem ist, dass, wenn das Instrument bewegt wird, also ein dynamischer Zustand vorliegt, aufgrund der Messrate des Systems der Plausibilitätswert ebenfalls steigt. Das heißt, dass die Bewegung ebenfalls als Störung wahrgenommen wird, was als Eigenstörung bezeichnet wird. Da mit einem zu großen Plausibilitätswert behaftete Lagemessdaten nicht einer Visualisierung zugeführt werden, kann eine rasche Bewegung des Instrumentes zur Folge haben, dass die Navigationsinformation für das bewegte Instrument nicht angezeigt wird. Dies ist unkomfortabel für den Anwender. Ein durch die Plausibilitätsprüfung erlangter Sicherheitsgewinn geht somit zu Lasten der Verfügbarkeit der Navigationsinformation. Diese Einbuße muss vermieden werden, um das System ergonomisch einsetzen zu können.

Da die Störung zu einem unbestimmten Zeitpunkt und mit nicht zuvor bekannter Auswirkung auf die gemessene Instrumentenposition auftreten kann, werden folgende Anforderungen an eine risikominimierende Fehlerbehandlung gestellt:
Die Störungen, die die Messung der Instrumentenposition nach statischer Fehlerkorrektur aktuell beeinflussen, müssen vom System erkannt und quantifiziert werden.

Die Fehlererkennung untersucht in jedem Messzyklus die Messwerte zur Laufzeit.

Die Fehlererkennung soll nicht durch ein Referenzsystem optischer oder mechanischer Art unterstützt werden, weil dadurch der Systemaufbau unergonomisch und kostenintensiv wird.

Die erkannte quantifizierte Störung soll hinreichend genau mit dem tatsächlich auftretenden Positionsmessfehler an dem Instrumentenarbeitspunkt korrelieren. Hinreichend genau bedeutet hier, dass dynamische Positionsmessfehler, die über einem Sicherheitswert von maximal 2 mm liegen, mit einer Wahrscheinlichkeit von 95 % erkannt werden.

Messwerte, die mit einem dynamischen Positionsmessfehler beaufschlagt sind, werden weiter verarbeitet. Die Weiterverarbeitung führt dazu, dass die Messwerte korrigiert werden oder für Navigation ausgeschlossen oder speziell gekennzeichnet zur Anzeige gebracht werden.

Zur Erfüllung dieser Anforderungen wird ein Konzept entworfen, das auf einer dynamischen Fehlererkennung basiert. Diese Fehlererkennung, im Folgenden als Plausibilitätsprüfung bezeichnet, wird im Folgenden näher beschrieben. Die darauf aufbauenden weiteren Komponenten des Konzeptes werden anschließend erläutert.

Die Grundidee hierbei ist, zwischen einer Eigenstörung und einer Fremdstörung, hervorgerufen durch ein weiteres Objekt, zu unterscheiden und diese Störklassen unterschiedlich zu behandeln. Die Klasse Fremdstörung wird dabei aus Gründen der Risikominimierung wie oben beschrieben behandelt. Das bedeutet, das es wird zumindest eine Fehlerkugel um den Instrumentenarbeitspunkt herum dargestellt wird. Die Klasse der Eigenstörung wird nochmals in Bewegungszustände unterteilt. Während der Anwendung des navigierten Instrumentes treten unterschiedliche Bewegungszustände auf. Mit jedem Bewegungszustand ist eine Genauigkeitsanforderung an die dargestellte Position verknüpft.

Die Tabelle 1 zeigt die definierten Zustandsklassen und die Tabelle 2 beschreibt die Anforderungen an die einzelnen Bewegungszustände qualitativ. Aus der jedem Bewegungszustand zugeordneten Genauigkeitsanforderung folgt, dass Störungen bei dynamischen Vorgängen, wie das Bewegen des Instrumentes im Arbeitsraum, weniger strikt behandelt werden müssen. Es heißt, dass der Schwellwert für die Risikoklasse bei dem Zustand Bewegen höher liegen kann. Somit werden diese Eigenstörungen nicht herausgefiltert.

**Tabelle 1**

| **Zustand** | **Zustandsbeschreibung** | **Beschreibung der Messsituation** |
|---|---|---|
| z = 1 | Fremdstörung | Die gemessene Instrumentenposition wird durch ein weiteres Objekt beeinflusst |
| z = 2 | Eigenstörung - Positionieren des Instruments | Die gemessene Instrumentenposition wird nicht oder durch unzureichende Kalibrierdaten beeinflusst. |
| z = 3 | Eigenstörung - Rotation des Instruments | Die gemessene Instrumentenposition durch die Rotationsbewegung beeinflusst. |
| z = 4 | Eigenstörung - Bewegen des Instruments | Die gemessene Instrumentenposition durch die Translationsbewegung beeinflusst. |

**Tabelle 2**

| **Bewegungszustand** | **Definition** | **Genauigkeitsanforderung** |
|---|---|---|
| Positionieren | Das Instrument wird an einer Position still gehalten oder mit geringer Geschwindigkeit bewegt oder rotiert. | Hoch - Der Anwender gleicht in diesem Zustand, die aktuelle Position des Instruments am Patienten mit der dargestellten Position am Bildschirm ab. Die Information bedarf einer geringen Messunsicherheit, da u. U. Abstände zu sensiblen Strukturen verdeutlicht werden sollen und das Instrument an dieser Position eine Arbeit verrichtet (z.B. Bohren). Das Instrument wird aktiv eingesetzt. |
| Rotation | Das Instrument wird geschwenkt oder um die eigne Achse gedreht. | Mittel - Der Anwender richtet das Instrument aus, um den Bewegungszustand *Positionieren* zu erreichen. Das Instrument wird in diesem Zustand noch nicht aktiv eingesetzt. |
| Bewegen | Das Instrument wird mit einer Geschwindigkeit > 5 mm/s bewegt (Translation). | Niedrig - Der Anwender bewegt das Instrument im Arbeitsraum. Durch die Darstellung am Bildschirm erhält er eine grobe Orientierung in der Anatomie. Aufgrund der Bewegung ist ein genauer visueller Abgleich mit den Bilddaten nicht möglich. In diesem Bewegungszustand wird der Eingriffsort lokalisiert. |

Somit ist ein Verfahren zum Erzeugen von Bilddaten für eine Darstellung eines Instrumentes, insbesondere eines medizinischen Instrumentes, auf einer Anzeigevorrichtung geschaffen, wobei das Verfahren die folgenden Schritte umfasst: Erfassen von Lagemessdaten von wenigstens einem an dem Instrument angeordneten Lagesensor, bestimmen eines Bewegungszustands des Instrumentes auf der Grundlage der erfassten Lagemessdaten, Filtern der erfassten Lagemessdaten auf der Grundlage des bestimmten Bewegungszustandes und Bereitstellen eines Bilddatensatzes für eine geänderte Darstellung des Instrumentes wenigstens auf der Grundlage der gefilterten Lagemessdaten, welcher konfiguriert ist, das Instrument darzustellen.

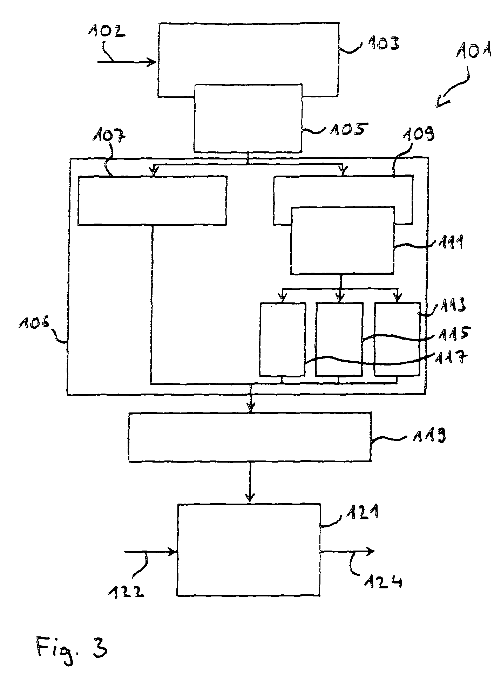

Fig. 3 veranschaulicht ein Konzept einer Umsetzung einer adaptiven Schwellwertvorgabe. Demgemäß ist zunächst ein Signalspeicher 103 vorgesehen, der benötigt wird, um einen Signalverlauf für eine nachfolgende statistische Analyse vorzuhalten. Es werden einem Eingang 102 zugeführte aktuelle Messwerte beispielsweise Positions- oder Lagedaten, p(k) sowie n vorherige Messwerte gespeichert. Ferner wird dem Eingang der aktueiie Plausibilitätswert d(k) zugeführt.

Ein Störungsklassifikator 105 schließt aus den gespeicherten Signalen auf die Art der Störung. Es wird eine Unterscheidung zwischen Fremdstörung und Eigenstörung getroffen. Keine erkennbaren Störungen werden ebenfalls als Eigenstörungen klassifiziert.

Somit wird bereits am Eingang einer Zustandsbestimmung 106 eine Trennung zwischen einer Fremdstörungsbehandlung 107 und einer Eigenstörungsbehandlung 109 vorgenommen.

Wurde durch den Störungsklassifikator 105 bestimmt, dass eine Eigenstörung vorliegt, so schließt ein Bewegungsklassifikator 111 aus dem Signal auf den aktuellen Bewegungszustand des Instrumentes. Dafür wurden zuvor die Signalverläufe für die einzelnen Zustände analysiert und Kriterien für die Klassifizierung herausgearbeitet, wie weiter unten gezeigt wird. Mit Hilfe dieser Kriterien kann die Klassifizierung zur Laufzeit vorgenommen werden. Es sind die Bewegungszustände Positionieren 117, Bewegen 115 und Rotieren 113 definiert. In Tabelle 2 sind diese Zustände genauer erläutert.

Der in der Zustandsermittlung 106 ermittelte Bewegungszustand, welcher die aktuelle Messsituation beschreibt, wird einer Schwellwertparametrisierung s = f(z) zugeführt, 119. Die Schwellwertparametrisierung s = f(z), 119, ordnet jedem ermittelten Bewegungszustand einen Schwellwert zu, welcher in einer nachfolgenden Stufe ausgewertet wird. Geeignete Schwellwerte wurden zuvor experimentell ermittelt. Der von der Schwellwertparametrisierung 119 ermittelte Schwellwert wird dem Schwellwertfilter 121 zugeführt. Der Schwellwertfilter 121 vergleicht den ihm über den Eingang 122 zugeführten aktuellen Plausibilitätswert p(k) mit dem parametrisierten Schwellwert s = f(z) für die Risikoklasse. Messwerte, die in die Risikoklasse fallen, werden gefiltert. Der Schwellwert wird fortlaufend an den aktuellen Bewegungszustand angepasst.

Der adaptive Schwellwertfilter erlaubt für den Fall, dass keine Fremdstörung erkannt wird, eine Anzeige der Navigationsinformation bei höherer Dynamik des Instrumentes. Gleichzeitig wird das Sicherheitskonzept nicht verletzt.

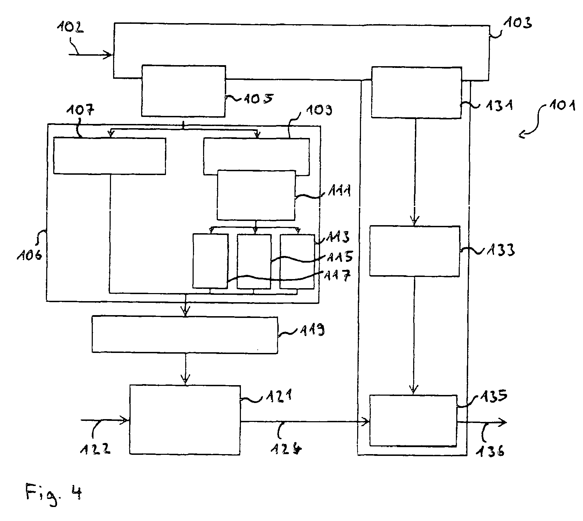

Die Fig. 4 veranschaulicht ein erweitertes Filterkonzept. Die oben beschriebene Behandlung dynamischer Fehler zielte ausschließlich auf das Herausfiltern, also das Verwerfen fehlerbehafteter Messwerte. Mit der Signalkonditionierung soll das Filterkonzept nochmals um eine Komponente erweitert werden, die dynamische Fehler, hervorgerufen durch Eigenstörung, korrigiert. Das Filter 101' liefert zu jedem Messwert, also zu gemessenen Lage- und/oder Postitionsdaten p(k) am Eingang 112 einen korrigierten Messwert p'(k) am Ausgang 136. Fig. 4 veranschaulicht die Funktionsweise des zu entwerfenden Filters. Die Blöcke 131, 133 und 135 gliedern sich in das bestehende Filterdesign zwischen dem Signalspeicher und dem eigentlichen Signalpfad ein. Die Vorverarbeitung kann parallel zu der Klassifizierung des Schwellwertfilters arbeiten. Die Komponente Signalkonditionierung 135, die die Positionsmessdaten manipuliert, wird im Signalpfad 124 hinter dem Schwellwertfilter 121 angeordnet. Die Datenrate im Signalpfad wird durch das Filterkonzept nicht verändert. Das heißt, dass die Verarbeitung der Signale in einem Messzyklus vorgenommen werden kann.

Die hinzugekommenen einzelnen Funktionsblöcke des erweiterten Filters sind eine Datenaufbereitung 131, welche die gespeicherten Signaldaten nutzt, um weitere Signale daraus zu generieren, beispielsweise Geschwindigkeit, Korrelation und/oder Varianz. In einem Modellvergleich 133 können die Signalverläufe analysiert und mit den Vorgaben eines Modells verglichen werden. Daraus leiten sich Parameter für die Komponente der Signalkonditionierung 135 ab. Die Signalkonditionierung 135 manipuliert die aktuell gemessenen und gefilterten Positionsmesswerte p(k). Der am Ausgang 136 anliegende Wert p'(k) ist somit eine Funktion von p(k) sowie zumindest des ermittelten Bewegungszustands.

Der Bewegungsklassifikator 111 bewertet die gespeicherten Signalverläufe hinsichtlich des Störungs- und Bewegungszustandes des Instrumentes. Dafür wurden zuvor in einer Untersuchung geeignete Kriterien für eine Klassifikation gefunden. In der Untersuchung wurden Signalverläufe der Instrumentenarbeitspunktverläufe in Abhängigkeit von den unterschiedlichen Lagesensordaten aufgezeichnet. Hierzu wurde jeweils eine bestimmte Bewegung, wie beispielsweise Translation, Rotation oder Positionieren durchgeführt. Die Signalverläufe wurden hinsichtlich eindeutiger Merkmale analysiert. Für die Signalanalyse wurden drei Größen eingeführt, mit denen die Signale charakterisiert werden können: Zunächst wurde der Korrelationskoeffizient eingeführt, welcher die Korrelation zwischen den beiden Instrumentenarbeitspunktsignalen für die gespeicherten Messwerte beschreibt. Als nächstes wurde die Memory-Varianz herangezogen, die ein Maß für die Streuung der gespeicherten Messwerte ist. Schließlich wurde eine Instant-Varianz herangezogen, welche ein Maß für die Streuung der letzten drei Messwerte ist. Dieses Signal folgt raschen Änderungen schneller als die Memory-Varianz. Die Varianz wurde eingeführt, weil bei einer Bewegung die Varianz höher ist als bei einem stillgehaltenem, also positioniertem, Instrument. Es wurde festgestellt, dass eine Translation nicht vorliegt, wenn eines der folgenden Kriterien zutrifft: Kriterium 1: die Korrelationskoeffizienten aller Freiheitsgrade (x, y, z) sind kleiner als ein experimentell ermittelter Wert; Kriterium 2: die Korrelationskoeffizienten von 2 Freiheitsgraden sind negativ; oder Kriterium 3: die Instant-Varianzen aller Freiheitsgrade sind kleiner als ein experimentell gefundener Wert. Hinsichtlich einer Rotation, die eine Schwenkbewegung des Instrumentes beschreibt, bei welcher der Instrumentenarbeitspunkt auf einer Position verbleibt, wurde festgestellt, dass eine Rotierbewegung nicht vorliegt, wenn das Kriterium 4 zutrifft, dass die Instant-Varianzen aller Freiheitsgrade kleiner als ein experimentell bestimmter Wert sind.

Ein durch einen Fremdkörper hervorgerufener Störungszustand liegt vor, wenn eines der folgenden Kriterien erfüllt wird: Kriterium 5: Die Memory-Varianzen aller Freiheitsgrade sind kleiner als ein experimentell bestimmter Wert; oder Kriterium 6: Die Korrelationskoeffizienten von genau zwei Freiheitsgraden sind kleiner als ein experimentell bestimmter Wert und der verbleibende Freiheitsgrad hat einen Korrelationskoeffizienten der kleiner ist als ein negativer experimentell bestimmter Wert.

Die drei Größen Korrelationskoeffizient, Instant-Varianz und Memory-Varianz werden zu jedem Messzyklus k mittels des Bewegungsklassifikators 111 aus dem Signalspeicher für die Freiheitsgrade beider Positionsmessungen berechnet.

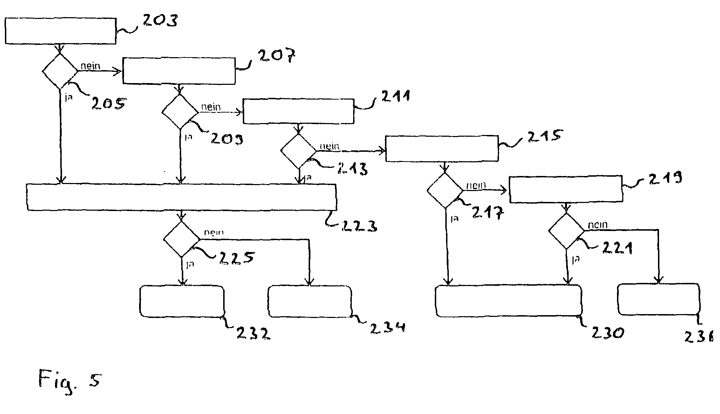

Die Fig. 5 veranschaulicht einen Algorithmus zur Klassifizierung, also zur Bestimmung eines Bewegungszustandes, auf der Grundlage der oben definierten Kriterien. Dabei werden die Kriterien sukzessiv geprüft und Entscheidungen getroffen. Als Ergebnis liefert der Algorithmus einen Bewegungszustand, der für die Parametrisierung des Schwellwertfilters herangezogen wird. Der in Fig. 5 dargestellte Klassifizierungsalgorithmus 201 prüft also die oben definierten einzelnen Kriterien und ermittelt schließlich einen Bewegungszustand des Instrumentes. In einem Schritt 203 überprüft der Algorithmus, ob das Kriterium 1 erfüllt ist, und verzweig in 205. In dem Falle, dass das Kriterium 1 nicht erfüllt ist, setzt der Algorithmus in 207 fort. In dem Falle, dass das Kriterium 1 erfüllt ist, setzt der Algorithmus in 223 fort. In 207 überprüft der Algorithmus das Kriterium 2, und verzweigt in 209. In dem Falle, dass das Kriterium 2 nicht erfüllt ist, setzt der Algorithmus in 211 fort. In dem Falle, dass das Kriterium 2 erfüllt ist, setzt der Algorithmus in 223 fort. In 211 prüft der Algorithmus das Kriterium 3, und verzweigt in 213. In dem Falle, dass das Kriterium 3 nicht erfüllt ist, setzt der Algorithmus in 215 fort. In dem Falle, dass das Kriterium 3 erfüllt ist, setzt der Algorithmus in 223 fort. In 215 prüft der Algorithmus das Kriterium 5, und verzweigt in 217. In dem Falle, dass das Kriterium 5 nicht erfüllt ist, setzt der Algorithmus in 219 fort. In dem Falle, dass das Kriterium 5 erfüllt ist, endet der Algorithmus in 230 und bestimmt den Bewegungszustand als Störungszustand, z = 1. In 219 überprüft der Algorithmus das Kriterium 6, und verzweigt in 221. In dem Falle, dass das Kriterium 6 nicht erfüllt ist, endet der Algorithmus in 236 und hat als Bewegungszustand die Translation, z = 4, bestimmt. In dem Falle, dass das Kriterium 6 erfüllt ist, endet der Algorithmus in 230 und bestimmt den Bewegungszustand als Störungszustand, z = 1. In 223 prüft der Algorithmus das Kriterium 4, und verzweigt in 225. In dem Falle, dass das Kriterium 4 nicht erfüllt ist, endet der Algorithmus in 234 und hat den Bewegungszustand als Rotieren bestimmt, z = 3. In dem Falle, dass das Kriterium 4 erfüllt ist, endet der Algorithmus in 232 und hat den Bewegungszustand als Positionieren bestimmt, z = 2. Jedem der ermittelten Bewegungszustände, also Störungszustand, Positionieren, Rotieren und Translation, ist ein Schwellwert zugeordnet. Wie in den Fig. 3 und 4 veranschaulicht ist, entscheidet ein Schwellwertfilter, ob der aktuelle Plausibilitätswert d einen der parametrisierten Schwellwerte überschreitet oder unterschreitet. In dem Falle, dass der ermittelte Schwellwert überschritten wird, wird der zugehörige Messwert verworfen, das heißt er wird in 124 nicht ausgegeben und steht somit nicht für eine Darstellung des Instruments auf der Anzeigevorrichtung zur Verfügung. In dem Falle, dass der Plausibilitätswert kleiner ist als der parametrisierte Schwellwert aus 119 wird der aktuelle Positionswert in 121 nach 124 durchgelassen. Die Zuordnung eines Bewegungszustandes aus dem Bewegungsklassifikator zu einem Schwellwert wird als Parametrisierung bezeichnet, welche als eine Tabelle für jeden Bewegungszustand hinterlegt ist. Zur Erstellung der Tabelle wurden die einzelnen Schwellwerte experimentell für das Instrument ermittelt. Die letzte Komponente im Signalpfad ist die Visualisierung der Positionsdaten, also eine Darstellung des Instrumentes auf einer Anzeigevorrichtung. Die korrigierten und gefilterten Daten werden in ein Bilddatenkoordinatensystem transformiert und angezeigt. Die Visualisierung umfasst folgende zusätzliche Eigenschaften: 1. Fehlerabschätzung: Die Messunsicherheit, die durch die Registrierung und Messstörungen besteht, wird abgeschätzt. Als Maß für die Registrierunsicherheit wird ein Gütewert aus dem Registrieralgorithmus verwendet. Der Wert gibt die maximale Abweichung der zueinander registrierten Punkte an. Dieser Wert wird vereinfacht richtungsunabhängig verwendet. Als Maß für fehlerbehaftete Positionsmesswerte dient der Plausibilitätswert d. Dieser wird in jedem Messzyklus k berechnet. 2. Visualisieren der Messunsicherheit: Die abgeschätzte Messunsicherheit wird als Raum um die dargestellte Position des Instrumentenarbeitspunktes in Form einer Kugel visualisiert. In orthogonalen Schnittansichten ist dies daher ein Kreis. Die Dynamik dieser Anzeige ermöglicht es einem Anwender, den Störungszustand der aktuellen Positionsmessung zu beurteilen. 3. Visualisierung des Störungszustandes: Messdaten, die durch den Schwellwertfilter verworfen wurden, werden nicht für die Navigationsanzeige verwendet. Dieser Zustand übermäßiger Störung wird dem Anwender durch eine rote Einfärbung des, in den Bilddaten repräsentierten, Instrumentes signalisiert. Das Instrument wird auf der letzten gültigen gemessenen Position dargestellt, bis ein neuer gültiger Messwert vorliegt.

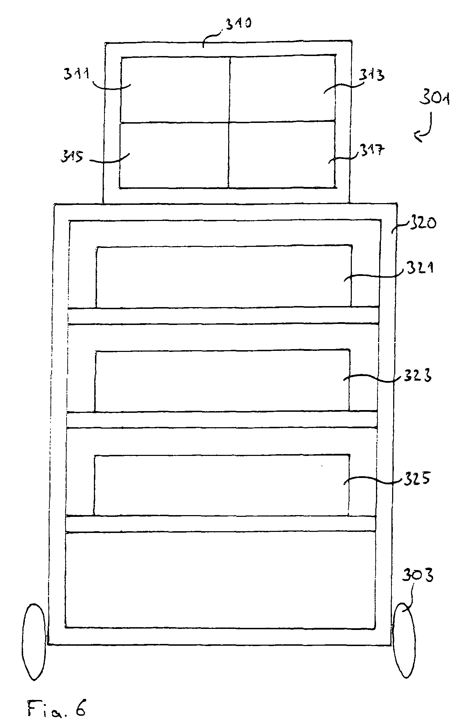

In der Fig. 6 ist ein Endoskopiesystem 301 veranschaulicht, das unabhängig von dem oben beschriebenen Verfahren, System, Computerprogrammprodukt, Computerprogramm und Datenträger bereitgestellt ist, aber in der Lage ist, mit dem oben beschriebenen Verfahren, System, Computerprogrammprodukt, Computerprogramm und Datenträger wirksam zusammenzuarbeiten. Das Endoskopiesystem 301 umfasst wenigstens einen Monitor 310 und wenigstens einen Navigationsmodul 321 und ist mittels Rollen oder Rädern 303 bequem transportierbar. Das Endoskopiesystem 301 ist dazu konfiguriert, wenigstens eine synchronisierte Video- und Navigationsinformation auf dem Monitor, beispielsweise einem Endoskopiemonitor bereitzustellen. Beispielsweise ist das Endoskopiesystem 301 dazu konfiguriert, eine auf der Grundlage des oben beschriebenen Verfahrens synchronisierte Video- und Navigationsinformation auf dem Monitor 310 darzustellen. Die Synchronisation umfasst beispielsweise eine Aktualisierung der, wie oben beschrieben, um den Instrumentenarbeitspunkt herum dargestellten, auf der Grundlage des Plausibilitätswertes und der erfassten Lagemessdaten bestimmten Kugel zur Veranschaulichung einer Messunsicherheit. Die Aktualisierung der Darstellung kann beispielsweise auf einer Aktualisierung des jeweils zur Anzeige gebrachten Bilddatensatzes beruhen. Vorteilhaft kann für eine gleichzeitige Visualisierung mehrerer Ansichten vorgesehen sein, den Monitor 310, im Betrieb, mittels einer geeigneten Ansteuerung des Monitors, in vier Teilansichten 311, 313, 315 und 317 zu unterteilen. Die Bezugszeichen 323 und 325 bezeichnen weitere am Endoskopieturm vorgesehene Standardmoduln.

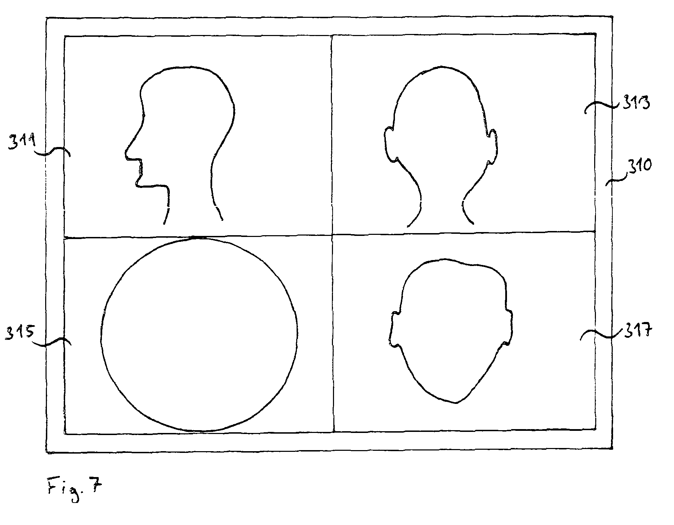

Die Fig. 7 stellt eine beispielhafte Darstellung von Endoskopieinformationen auf einem Endoskopiemonitor 310 dar. Der beispielsweise mittels geeigneter Monitoransteuerung in vier Teilansichten aufgeteilte Monitor 310 stellt in einer Teilansicht 311 einen sagittalen, in einer Teilansicht 313 einen coronaren und in Teilansicht 317 einen axialen Schnitt durch einen Kopf eines Patienten dar. In einer Teilansicht 315 können beispielsweise synchronisierte Video- und/oder Navigationsinformationen dargestellt werden. Diese Informationen können beispielsweise nach dem oben beschriebenen Verfahren berechnet und/oder aufbereitet worden sein.

Mit anderen Worten ist mittels des beschriebenen Endoskopiesystems 301 ein Dokumentations- und Behandlungsmodul für eine Navigation in der Medizin bereitgestellt. Das System zur Dokumentation und Durchführung von navigierten Eingriffen besteht zumindest aus einem Navigationsmodul 321, das als zusätzliche Komponente eines herkömmlichen Videoendoskopieturms bereitgestellt sein kann. Das System umfasst vorzugsweise wenigstens einen Monitor. Vorzugsweise ist dieses System nicht als zusätzliches mobiles oder stationäres System konfiguriert, sondern in dem Videoendoskopieturm integriert. Die aufgenommenen Videodaten können zusammen mit Navigationsdaten, die beispielsweise wie oben beschrieben synchronisiert sind, zu Dokumentationszwecken aufgezeichnet werden. Ferner umfasst das Modul vorzugsweise Schnittstellen für optische und / oder magnetische Navigationssensoren. Vorteilhaft ist es bei diesem System während einer Bedienung und / oder während eines Arbeitens mit dem System nicht erforderlich, Eingaben an dem System vorzunehmen. Dadurch ist eine Bedienperson entlastet und kann ihre Aufmerksamkeit vollständig auf die Arbeit an einer Umgebung des Instrumentenarbeitspunktes richten. Wenigstens ein elektromagnetischer Navigationssensor kann in einer festgelegten Ausrichtung an einem Operationstisch angebracht werden und mit dem Navigationsmodul 321 derart verbunden werden, dass die Sensordaten des elektromagnetischen Navigationssensors vom Navigationsmodul 321 ausgelesen und ausgewertet werden können und anschließend auf dem Monitor dargestellt werden können. Wenigstens ein optischer Navigationssensor kann an einem selbsthaltenden Kugelgelenk befestigt und mit einer Hand einer Bedienperson ausgerichtet werden. Der optische Navigationssensor kann beispielsweise als Kamera ausgeführt sein. Der optische Navigationssensor kann derart mit dem Navigationsmodul 321 verbunden sein, dass die Sensordaten des elektromagnetischen Navigationssensors vom Navigationsmodul 321 ausgelesen und ausgewertet werden können und anschließend auf dem Monitor dargestellt werden können. Vorzugsweise ist das System derart konfiguriert, dass die Navigationsinformationen und die Videoinformationen auf zwei Monitore aufgeteilt und dargestellt werden können. Es kann beispielsweise vorgesehen sein, dass das Navigationsmodul über eine drahtlose Verbindung mit dem Navigationssensor kommuniziert. Eine derartige Verbindung könnte beispielsweise über ein Bluetooth-Protokoll bereitgestellt sein. Es kann beispielsweise ferner vorgesehen sein, dass das Navigationsmodul Patientenbilddaten über eine drahtlose Verbindung bezieht. Beispielsweise könnte über ein WLAN im Gebäude ein Zugriff auf eine Patientendatenbank eines zentralen Servers erfolgen. Das vorgestellte System kann in geeigneter Ausführung einen oder mehrere der folgenden Vorteile bereitstellen: Das Navigationssystem kann als ein Modul für einen Endoskopieturm bereitgestellt sein, so dass eine Handhabbarkeit verbessert ist. Das Navigationssystem kann vollständig in dem modularen Endoskopieturm integriert sein. Ferner kann es kompatible Schnittstellen zu weiteren gängigen Endoskopiemoduln bereitstellen. Ein klinischer Arbeitsablauf wird deutlich verbessert, da kein zusätzliches Gerät erforderlich ist. Der optische Navigationssensor, der beispielsweise als Kamera ausgeführt ist, ist separat vom Endoskopieturm auf einem Stativ positionierbar. Die Kameraschnittstelle ist derart konfiguriert, dass sie alle gängigen Navigationskameras unterstützt. Das Navigationssystem ist derart konfiguriert, dass es eine Videoeinblendung vornehmen kann, also, dass verschiedene bestimmte Bilddaten beispielsweise auf einem Bildschirmbereich in überlagerter Weise dargestellt werden, so dass es einem Bediener beispielsweise möglich ist, reale Bilddaten und bewertete und ausgewertete Sensordaten gleichzeitig und mit räumlicher Zuordnung zu erfassen und auf diese Weise während der Arbeit eine verbesserte Navigation des Instruments vornehmen zu können. Schließlich ist das beschriebene System in der Lage, Videodaten und Schichtbilddaten zu dokumentieren, also beispielsweise die Daten als momentane Standbilddaten oder als Datenströme mit einem Zeitstempel zu versehen und zu speichern.

## Patentansprüche

1. Verfahren zum Erzeugen von Positionsdaten eines Instrumentes (1), wobei das Verfahren die folgenden Schritte umfasst:
- Erfassen von Lagemessdaten von wenigstens zwei an dem Instrument (1) angeordneten Lagesensoren (7, 9),
- Bestimmen eines Plausibilitätswertes, auf der Grundlage der Lagemessdaten, die unter Verwendung von den wenigstens zwei an dem Instrument angeordneten Lagesensoren erfasst wurden,
- Klassifizieren eines Bewegungszustands des Instruments (1) auf der Grundlage aktuell erfasster und in der Vergangenheit erfasster Lagemessdaten, wobei jedem klassifizierten Bewegungszustand des Instruments ein Plausibilitätsschwellwert zugeordnet ist,
Zuordnen eines Plausibilitätsschwellwerts zu dem klassifizierten Bewegungszustand, so dass der Plausibilitätsschwellwert fortlaufend an den klassifizierten Bewegungszustand angepasst wird,
- Filtern der erfassten Lagemessdaten auf der Grundlage eines Vergleichs zwischen dem bestimmten Plausibilitätswert und dem zugeordneten Plausibilitätsschwellwert,
- Bereitstellen eines Bilddatensatzes für eine Darstellung des Instruments (1) auf einer Anzeigevorrichtung wenigstens auf der Grundlage der gefilterten Lagemessdaten, welcher konfiguriert ist, das Instrument (1) auf der Anzeigevorrichtung darzustellen., wobei der Bilddatensatz ein Bilddatensatz aus wenigstens einem Verfahren der Gruppe ist, welche umfasst: Computertomographie, digitale Volumentomographie, Magnetresonanztomographie, Sonographie, Duplexuntersuchung, Angiographie, nuklearmedizinische Bildgebungsverfahren, mit Kontrastmittelgabe einhergehende medizinische Bildgebungsverfahren, Durchleuchtungsverfahren und radiologische Additions- oder Subtraktionsverfahren, und
- Bereitstellen des Bilddatensatzes für eine geänderte Darstellung des Instruments (1) wenigstens auf der Grundlage der gefilterten Lagemessdaten, welcher konfiguriert ist, das Instrument (1) darzustellen.

2. Verfahren nach Anspruch 1, **gekennzeichnet durch** die Schritte:
- Zuordnung mindestens einer Koordinatentransformation von dem Lagesensor (7, 9) zu wenigstens einem Instrumentenarbeitspunkt zu jedem Lagesensor (7, 9); und
- Bestimmung des Instrumentenarbeitspunktes **durch** mathematische Verknüpfung von Lagemessdaten eines Lagesensors (7, 9) und zugeordneter Koordinatentransformation.

3. Verfahren nach einem der vorangehenden Ansprüche, **dadurch gekennzeichnet, dass** die erfassten Lagemessdaten auf der Grundlage wenigstens einer aktuellen Lagemessung gebildet werden.

4. Verfahren nach einem der vorangehenden Ansprüche, **dadurch gekennzeichnet, dass** Kriterien zur Unterscheidung von Bewegungszuständen miteinander logisch verknüpft sind.

5. Verfahren nach einem der vorangehenden Ansprüche, **dadurch gekennzeichnet, dass** das Filtern ein Akzeptieren der aktuellen Lagemessdaten für den Fall, dass der Plausibilitätswert kleiner oder gleich dem Plausibilitätsschwellwert ist, und ein Verwerfen der aktuellen Lagemessdaten für den Fall, dass der Plausibilitätswert größer als der Plausibilitätsschwellwert ist umfasst.

6. Verfahren nach der vorangehenden Ansprüche, **dadurch gekennzeichnet, dass** auf der Grundlage einer logischen Verknüpfung von Kriterien bestimmt wird, ob oder ob nicht eine Eigenstörung und / oder eine Fremdstörung der erfassten Lagemessdaten vorliegt.

7. System zur Darstellung eines Instruments (1), auf einer Anzeigevorrichtung, wobei das System umfasst:
- wenigstens zwei an dem Instrument (1) angeordnete Lagesensoren (7, 9) zum Erfassen
von Lagemessdaten des Instruments (1),
- eine Datenverarbeitungseinrichtung, die dazu konfiguriert ist, das Verfahren nach einem der Ansprüche 1 bis 6 durchzuführen, und eine Anzeigevorrichtung

8. System nach Anspruch 7, **gekennzeichnet durch** eine Datenverarbeitungseinrichtung, die dazu konfiguriert ist, einen auf der Grundlage eines Verfahrens nach einem der Ansprüche 1 bis 6 bestimmten Bilddatensatz darzustellen.

9. System nach Anspruch 7 oder 8 **dadurch gekennzeichnet, dass**
wenigstens ein erster Lagesensor (7, 9) an einem distalen und wenigstens ein zweiter Lagesensor (7, 9) an einem proximalen Ende des Instruments (1) angeordnet ist.

10. Computerprogramm-Produkt mit Programmcode, der dazu konfiguriert ist, das Verfahren nach einem der Ansprüche 1 bis 6 auszuführen, wenn der Programmcode auf einem Computer ausgeführt wird.

## Claims

1. A method for generating position data of an instrument (1), wherein the method encompasses the following steps:
- capturing position measurement data of at least two position sensors (7, 9) which are arranged at or on the instrument (1),
- determining a plausibility value on the basis of the position measurement data which were captured using the at least two position sensors which are arranged at or on the instrument,
- classifying a movement state of the instrument (1) on the basis of currently captured position measurement data and such which were detected in the past, wherein a plausibility threshold value is allocated to each classified movement state of the instrument,
- allocating a plausibility threshold value to the classified movement state, so that the plausibility threshold value is continuously adapted to the classified movement state,
- filtering the captured position measurement data on the basis of a comparison between the determined plausibility value and the allocated plausibility threshold value ,
- providing an image data set for displaying or presenting the instrument (1) on a display apparatus at least on the basis of the filtered position measurement data, which is configured to display the instrument (1) on the display apparatus, wherein the image data set is an image data set from at least one of the methods out of the group comprising: computer tomography, digital volume tomography, magnetic resonance tomography, sonography, duplex examination, angiography, nuclear medical imaging, medical imaging using administration of a contrast agent, radiography and radiological addition or subtraction methods, and
- providing the image data set for an amended display or presentation of the instrument (1) at least on the basis of the filtered position measurement data, which is configured to display the instrument (1).

2. The method according to claim 1, **characterized in** the steps:
- allocating at least one coordinate transformation of the position sensor (7, 9) to at least one operating point of the instrument to each position sensor (7, 9), and
- determining the operating point of the instrument by mathematical combination of position measurement data of a position sensor (7, 9) and allocated coordinate transformation.

3. The method according to one of the preceding claims, **characterized in that** the captured position measurement data is generated on the basis of at least one current position measurement.

4. The method according to one of the preceding claims, **characterized in that** criteria to differentiate movement states are logically combined with each other.

5. The method according to one of the preceding claims, **characterized in that** filtering encompasses accepting the current position measurement data for the case that the plausibility value is smaller than or equal to the plausibility threshold value, and dismissing the current position measurement data for the case that the plausibility value is greater than the plausibility threshold value.

6. The method according to one of the preceding claims, **characterized in that** it is determined on the basis of a logical combination of criteria whether a self-interference and/or an external interference of the captured position measurement data exists or not.

7. A system for displaying or presenting an instrument (1) on a display apparatus, wherein the system encompasses:
- at least two position sensors (7, 9) for capturing position measurement data of the instrument (1), the position sensors being arranged at or on the instrument (1),
- a data processing device which is configured to execute the method according to one of the claims 1 to 6, and
- a display apparatus.

8. The system according to claim 7, **characterized by** a data processing device which is configured to present or display a particular image data set which is determined on the basis of a method according to one of the claims 1 to 6.

9. The system according to claim 7 or 8, **characterized in that** at least a first position sensor (7, 9) is arranged at or on a distal end and at least a second position sensor (7, 9) is arranged at or on a proximal end of the instrument (1).

10. A computer program product with a program code which is configured for executing the method according to one of the claims 1 to 6 when the computer program is executed on a computer.

## Revendications

1. Un procédé pour générer des données de position d'un instrument (1), le procédé comprenant les étapes suivantes :
- saisir des données de mesure de position d'au moins deux capteurs de position (7, 9) disposés sur l'instrument (1),
- déterminer une valeur de plausibilité sur la base des données de mesure de position ayant été saisies par l'usage d'au moins des deux capteurs de position disposés sur l'instrument,
- classifier un état de mouvement de l'instrument (1) sur la base de données saisies de mesure de position actuelles et antérieures, une valeur de seuil de plausibilité étant affectée à chaque état de mouvement classifié,
- affecter une valeur de seuil de plausibilité à l'état de mouvement, de sorte que la valeur de seuil de plausibilité soit adaptée de manière continue à l'état de mouvement classifié,
- filtrer les données saisies de mesure de position sur la base d'une comparaison entre la valeur de plausibilité déterminée et la valeur seuil de plausibilité affectée,
- fournir un ensemble de données d'image pour une représentation de l'instrument (1) sur un dispositif d'affichage sur la base au moins des données filtrées de mesure de position lequel est configuré pour représenter l'instrument (1) sur le dispositif d'affichage, l'ensemble de données d'image étant un ensemble de données d'image d'au moins un procédé du groupe comprenant : la tomographie assistée par ordinateur, la tomographie volumétrique numérique, la tomographie à résonance magnétique, l'échographie, les examens en mode duplex, l'angiographie, les procédés d'imagerie de médecine nucléaire, les procédés d'imagerie accompagnés de l'addition de produit de contraste, les procédures aux rayons X et les procédés d'addition ou de soustraction radiologiques, et
- fournir l'ensemble de données d'image pour une représentation modifiée de l'instrument (1) sur la base au moins des données filtrées de mesure de position lequel est configuré pour représenter l'instrument (1).

2. Procédé selon la première revendication **caractérisé par** les étapes :
- affectation d'au moins une transformation de coordonnées du capteur de position (7, 9) à au moins un point de fonctionnement de l'instrument de chaque capteur de position (7, 9) ; et
- détermination du point de fonctionnement au moyen d'un lien mathématique entre les données de mesure de position d'un capteur de position (7, 9) et de la transformation de coordonnées correspondante.

3. Procédé selon l'une des revendications précédentes **caractérisé en ce que** les données saisies de mesure de position sont formées sur la base d'au moins une mesure de position actuelle.

4. Procédé selon l'une des revendications précédentes **caractérisé en ce que** des critères pour différencier les états de mouvement sont combinés logiquement les uns avec les autres.

5. Procédé selon l'une des revendications précédentes **caractérisé en ce que** le filtrage comprend l'acceptation des données de mesure de position actuelles pour le cas où la valeur de plausibilité est plus petite ou égale à la valeur de seuil de plausibilité et le rejet des données de mesure de position actuelles pour le cas où la valeur de plausibilité est plus grande que la valeur de seuil de plausibilité.

6. Procédé selon l'une des revendications précédentes **caractérisé en ce qu'**il est déterminé sur la base d'un lien logique de critères si une auto-interférence et/ou un signal parasite des données de mesure de position existe.

7. Système pour la représentation d'un instrument (1) sur un dispositif d'affichage, le système comprenant :
- au moins deux capteurs de position (7, 9) disposés sur l'instrument (1) pour saisir des données de mesure de position de l'instrument (1),
- un moyen de traitement des données configuré pour exécuter un procédé selon l'une de revendications 1 à 6, et
- un dispositif d'affichage.

8. Système selon la revendication 7 **caractérisé par** un moyen de traitement des données configuré pour représenter un ensemble de données d'image obtenu sur la base d'un procédé selon l'une des revendications 1 à 6.

9. Système selon l'une des revendications 7 ou 8 **caractérisé en ce qu'**au moins un premier capteur de position (7, 9) est disposé sur une extrémité distale et au moins un second capteur de position (7, 9) est disposé sur une extrémité proximale de l'instrument (1).

10. Un produit logiciel avec un code de programme configuré pour exécuter le procédé selon l'une des revendications 1 à 6, lors de l'exécution du code de programme sur un ordinateur.
